# EUROPÄISCHE PATENTSCHRIFT

(11) **EP 0 514 863 B1**
(45) Veröffentlichungstag und Bekanntmachung des Hinweises auf die Patenterteilung: **13.09.1995**
(21) Anmeldenummer: 92108508.0
(22) Anmeldetag: 20.05.1992
(51) Int. Cl.: C07C 63/68, C07C 51/377, C07C 51/56

(54) **Verfahren zur Herstellung von 3,4,6-Trifluorphthalsäure und deren Anhydrid**
Process for preparing 3,4,6-trifluorophthalic acid and its anhydride
Procédé de préparation d'acide 3,4,6-trifluorophtalique et de son anhydride

(30) Priorität: 22.05.1991 DE 4116625
(43) Veröffentlichungstag der Anmeldung: 25.11.1992
(73) Patentinhaber: HOECHST AKTIENGESELLSCHAFT, 65926 Frankfurt am Main (DE)
(72) Erfinder: Papenfuhs, Theodor, Dr., W-6000 Frankfurt am Main (DE); Pfirmann, Ralf, Dr., W-6103 Griesheim (DE)

(56) Entgegenhaltungen:
- PATENT ABSTRACTS OF JAPAN vol. 10, no. 131 (C-346)(2188) 15. Mai 1986 & JP-A-60 258 143
- PATENT ABSTRACTS OF JAPAN vol. 13, no. 423 (C-638)(3771) 20. September 1989 & JP-A-01 160 944
- SYNLETT Juni 1990, STUTTGART,DE Seiten 339 - 340 N J O'REILLY ET AL 'Selective hydrodechlorination of 3,4,5,6-tetrachlorophthalic anhydride: Preparation of3,4,6-trichlorophthalic acid'
- SYNLETT Oktober 1990, STUTTGART,DE Seiten 609 - 610 N J O'REILLY ET AL 'An expedient route to the quinolone antibacterial intermediate,2,4,5-trifluorobenzoic acid'

## Beschreibung

Die vorliegende Erfindung betrifft ein verbessertes Verfahren zur Herstellung von 3,4,6-Trifluorphthalsäure bzw. deren Anhydrid, die wertvolle Zwischenprodukte zur Herstellung hochwirksamer antibakterieller Mittel darstellen. So kann die 3,4,6-Trifluorphthalsäure durch Decarboxylierung (US 4 935 541, Occidental Chem. Corp., 19.6.90; JP 01 160 944, Nippon Carbide Industries Co., Inc., 23.6.89; JP 01 052 737, Nippon Carbide Industries Co., Inc., 28.2.89; JP 01 025 737, Nippon Carbide Industries Co., Inc., 27.1.89) nach verschiedenen Methoden in 2,4,5-Trifluorbenzoesäure überführt werden, die ihrerseits nach literaturbekannten Verfahren (PCT Int. Appln. WO 8906649, Warner-Lambert Co., 27.7.89; J. Med. Chem. 31 (5), 983-991 (1988), Warner-Lambert Co.; EP 227088, Abbott Laboratories, 1.7.87; EP 164619, Bayer AG, 12.10.88; DE 36 00 891, Bayer AG, 16.7.87; DE 34 20 743, Bayer AG, 5.12.85; EP 191185, Daiichi Seiyaku Co., Ltd., 20.8.86; JP 60 072 885, Daiichi Seiyaku Co., Ltd., 20.4.85) zu Chinoloncarbonsäurederivaten umgesetzt werden kann. Neben 2,3,4,5-Tetrafluorbenzoesäure stellt 2,4,5-Trifluorbenzoesäure den wichtigsten Grundstoff für die Herstellung derartiger antibakterieller Mittel dar.

Es ist bekannt, 3,4,6-Trifluorphthalsäure durch reduktive Dehalogenierung von 3,4,5,6-Tetrafluorphthalodinitril in saurer Lösung mittels metallischem Zink herzustellen (JP 01 160 944 A2; Nippon Carbide Industries Co., Inc., 23.6.89). Bei diesem Verfahren sind aber Werkstoffprobleme durch Fluorwasserstoffkorrosion der technischen Durchführbarkeit abträglich. Ferner führt die Verwendung von Zink in saurer Lösung bei erhöhten Temperaturen zu schneller Wasserstoffentwicklung, wobei zum einen Sicherheitsprobleme auftreten, zum anderen größere Zinkmengen zur Dehalogenierung benötigt werden.

Außerdem kann 3,4,6-Trifluorphthalsäure durch saure Hydrolyse von N-Alkyl- oder Aryltrifluorphthalimiden (N. J. O'Reilly, W.S. Derwin, L.B. Fertel, H.C. Lin, Synlett (1990), 609-610) hergestellt werden, die ihrerseits aus den entsprechenden Trichlorverbindungen durch Halex-Reaktion erhalten werden können. Bei der Hydrolyse werden die Alkyl- oder Arylammoniumverbindungen freigesetzt, die entweder zurückgewonnen oder aus dem Abwasser durch Reinigung eliminiert werden müssen. Diese Nachteile werden beim erfindungsgemäßen Verfahren vermieden. Das erfindungsgemäße Verfahren zur Herstellung von 3,4,6-Trifluorphthalsäure hat gegenüber den bekannte Verfahren zusätzlich den Vorteil, daß auf Tetrafluorphthalsäureanhydrid als Ausgangsverbindung zurückgegriffen werden kann.

Es wurde nun gefunden, daß man 3,4,6-Trifluorphthalsäure der Formel (1) 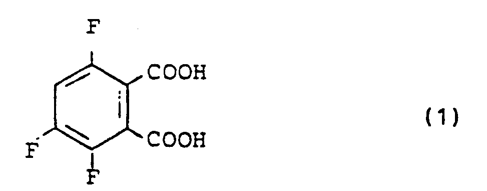
bzw. deren Anhydrid in guten Ausbeuten und in vorteilhafter Weise herstellen kann, indem man Tetrafluorphthalsäureanhydrid der Formel (2) 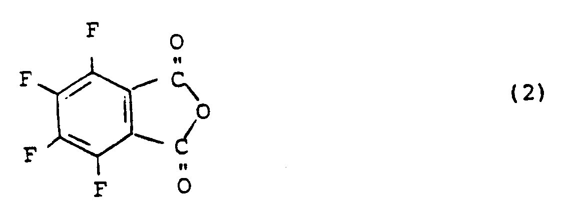
oder Tetrafluorphthalsäure in wäßrig-alkalischem Medium mit Zink, vorzugsweise Zinkstaub, bei Temperaturen von 20°C bis 160°C, vorzugsweise von 80°C bis 110°C, dehalogeniert und gegebenenfalls die erhaltene 3,4,6-Trifluorphthalsäure durch Entwässern in bekannter Weise in das Anhydrid überführt.

Das wäßrig-alkalische Reaktionsmedium wird hergestellt durch Lösen der Alkalimetallhydroxide, wie beispielsweise Lithiumhydroxid, Natriumhydroxid oder Kaliumhydroxid, der Erdalkalimetallhydroxide, wie beispielsweise Magnesiumhydroxid, Calciumhydroxid, Strontiumhydroxid oder Bariumhydroxid, sowie der entsprechenden Carbonate, Hydrogencarbonate oder Phosphate in Wasser, soweit diese alkalisch wirkende Lösungen, wie nachfolgend beschrieben, ergeben. Diese alkalisch wirkenden Verbindungen werden dabei in solchen Mengen eingesetzt, daß der pH-Wert des wäßrigen Reaktionsmediums bei Reaktionstemperatur 7 bis 15, vorzugsweise etwa 11 bis etwa 14, beträgt.

Ein Bodenkörper dieser alkalisch wirkenden Verbindungen aufgrund deren Schwerlöslichkeit im Reaktionsmedium kann ohne weiteres toleriert werden.

Die eingesetzten Zinkmengen bewegen sich zwischen 100 und 500 mol.-%, vorzugsweise zwischen 200 und 300 mol.-%, bezogen auf Tetrafluorphthalsäureanhydrid bzw. Tetrafluorphthalsäure.

Die Überführung der verfahrensgemäß erhaltenen 3,4,6-Trifluorphthalsäure in das Anhydrid kann entweder durch Entwässern einer Schmelze von 3,4,6-Trifluorphthalsäure im Vakuum oder analog der Herstellung der entsprechenden Trichlorverbindung durch azeotrope Entwässerung in siedendem Xylol (Lit. sh. Synlett (1990), 339-340, unten zitiert) erfolgen. Wie dies bei mehrfach halogenierten Phthalsäuren literaturbekannt ist, können diese sehr leicht entwässert werden (Delbridge, J. Am. Chem. Soc. 41, 400-409 (1919); zum Teil wandeln diese sich schon beim Schmelzen in das Anhydrid um. Somit kommt eine Herstellungsmethode für die Phthalsäure quasi auch einer Herstellungsmethode für das Anhydrid gleich. Umgekehrt wandelt sich das Anhydrid bereits beim Eintragen in Wasser in die Phthalsäure um.

Das erfindungsgemäße Verfahren kann bei Atmosphärendruck, Überdruck oder Unterdruck durchgeführt werden.

Wenn auch eine Dehalogenierung mittels Zinkstaub in wäßrig-alkalischem Medium von der Tetrachlorphthalsäure bekannt ist (Neil J. O'Reilly, William S. Derwin, Lawrence B. Fertel, Henry C. Lin, Synlett (1990) 339-340), so war es dennoch überraschend, daß auf ähnliche Weise in wäßrig-alkalischem Medium auch Fluorverbindungen dehalogeniert werden können. In wäßrig-saurer Lösung ist die reduktive Defluorierung (JP 01 160 944, Nippon Carbide Ind. Co., Inc., 23.6.89) von Tetrafluorphthalodinitril bekannt. Das hohe Reduktionspotential des Zinks in saurer Lösung bedingt hier die hohe Reaktionsgeschwindigkeit. Es ist bekannt, daß die Reaktivität bei der Dehalogenierung der Reihe F<<Cl<Br<J folgt (vgl. Houben-Weyl, Methoden der organischen Chemie, Band IV/1c, Seiten 364-370). Es war ferner überraschend, daß mit sehr hoher Selektivität ein Fluoratom in der 4-Stellung zur Carboxylgruppe entfernt wird, wie durch ¹H- und ¹⁹F-NMR-Spektroskopie gezeigt werden konnte. Diese Selektivität war aufgrund der sehr hohen Aktivierung aller Fluoratome durch Anwesenheit von 6 stark elektronenziehenden Substituenten nicht mehr zu erwarten. Es bestand weiterhin die Gefahr einer aromatischen nucleophilen Substitution von Fluor gegen Hydroxid, die aber Überraschenderweise nicht oder in nur sehr geringem Umfange eintrat.

Durch das nachstehende Beispiel wird die Erfindung näher erläutert, ohne darauf beschränkt zu werden.

### Beispiel

Unter Stickstoff wurde zu einer Lösung von 3,58 g (89,5 mmol) Natriumhydroxid in 30 g Wasser 5,5 g (25 mmol) Tetrafluorphthalsäureanhydrid gegeben. Dann wurde auf 100°C erhitzt und 6,54 g (0,1 mol) Zinkstaub zugegeben. Der Reaktionsverlauf wurde durch GC(DC) verfolgt, nach 12 h war die Umsetzung abgeschlossen. Man versetzte mit je 50 ml Wasser und Essigester und saugte die voluminösen Feststoffe ab, die viermal mit je 50 ml Wasser gewaschen wurden. Das farblose Filtrat wurde auf pH 1 gebracht und mit Essigester extrahiert. Trocknen der organischen Phasen über MgSO₄, Filtration und Entfernen des Lösungsmittels im Vakuum ergab 4,72 g (22,2 mmol; 89 %) farbloses Pulver, das als 3,4,6-Trifluorphthalsäure identifiziert werden konnte (Schmelzpunkt 156-160°C). Azeotrope Entwässerung in siedendem Xylol lieferte das 3,4,6-Trifluorphthalsäureanhydrid.

### 3,4,6-Trifluorphthalsäure:

¹⁹F-NMR (DMSO-d₆, CFCl₃):
δ = -113.4 (B, F-6) (ddd, 1F, J_{AB} = 9,7 Hz, J_{BC} = 6.9 Hz, J_{BD} = 15.2 Hz)
δ = -128.6 (C, F-4) (ddd, 1F, J_{AC} = 10.3 Hz, J_{BC} = 6.9 Hz, J_{CD} = 22.9 Hz)
δ = -143.6 (D, F-3) (ddd, 1F, J_{AD} = 6.4 Hz, J_{BD} = 15.2 Hz, J_{CD} = 22.9 Hz)
¹H-NMR (DMSO-d₆, TMS):
δ = 7.80 (A, H-5) (ddd, 1H, J_{AB} = 9.7 Hz, J_{AC} = 10.3 Hz, J_{AD} = 6.4 Hz)
MS: m/z [%] = 55 (2.0), 61 (8.1), 80 (24.5) 99 (12.1), 111 (3.2), 130 (100), 158 (60.3), 202 ((M-H₂O)⁺, 32.2)

### Verwendet man anstelle von 3,58 g (89,5 mmol) Natriumhydroxid

15,21 g (40 mmol) Natriumphosphattrihydrat,
16,80 g (200 mmol) Natriumhydrogencarbonat,
5,02 g (89,5 mmol) Kaliumhydroxid,
13,82 g (0,1 mol) Kaliumcarbonat,
3,76 g (89,5 mmol) Lithiumhydroxidhydrat,
8,89 g (0,12 mol) Calciumhydroxid,
15,01 g (0,15 mol) Calciumcarbonat,
16,33 g (0,12 mol) Calciumhydrogenphosphatdihydrat,
7,57 g (40 mmol) Bariumhydroxidhydrat,
14,57 g (30 mmol) Magnesiumhydroxidcarbonatpentahydrat
und verfährt im übrigen wie vorstehend beschrieben, so gelangt man zum gleichen Ergebnis.

## Patentansprüche

1. Verfahren zur Herstellung von 3,4,6-Trifluorphthalsäure der Formel (1) 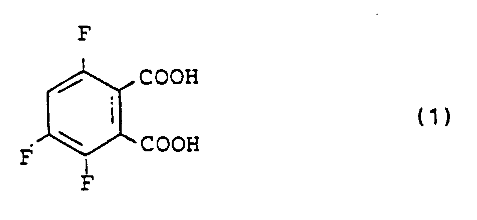 bzw. deren Anhydrid, dadurch gekennzeichnet, daß man
Tetrafluorphthalsäureanhydrid der Formel (2) 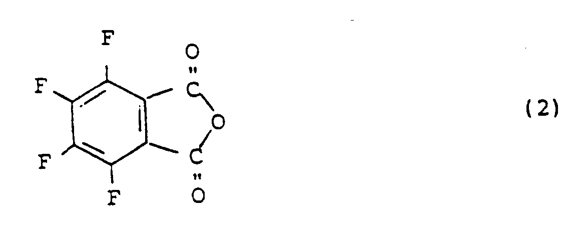 oder Tetrafluorphthalsäure in wäßrig-alkalischem Medium mit Zink bei Temperaturen von 20°C bis 160°C dehalogeniert und gegebenenfalls die erhaltene 3,4,6-Trifluorphthalsäure durch Entwässern in bekannter Weise in das Anhydrid überführt.

2. Verfahren nach Anspruch 1, dadurch gekennzeichnet, daß man bei Temperaturen von 80°C bis 110°C dehalogeniert.

3. Verfahren nach mindestens einem der Ansprüche 1 und 2, dadurch gekennzeichnet, daß man zur Einstellung des wäßrig-alkalischen Reaktionsmediums Alkalimetall- oder Erdalkalimetallhydroxide, -carbonate, - hydrogencarbonate oder -phosphate verwendet.

4. Verfahren nach mindestens einem der Ansprüche 1 - 3, dadurch gekennzeichnet, daß man die Alkalimetall- oder Erdalkalimetallhydroxide, - carbonate, -hydrogencarbonate oder -phosphate in solchen Mengen einsetzt, daß der pH-Wert im wäßrigen Reaktionsmedium 7 bis 15 beträgt.

5. Verfahren nach mindestens einem der Ansprüche 1 - 4, dadurch gekennzeichnet, daß man die Alkalimetall- oder Erdalkalimetallhydroxide, - carbonate, -hydrogencarbonate oder -phosphate in solchen Mengen einsetzt, daß der pH-Wert des wäßrigen Reaktionsmediums 11 bis 14 beträgt.

6. Verfahren nach mindestens einem der Ansprüche 1 - 5, dadurch gekennzeichnet, daß man 100 bis 500 mol.-% Zink, bezogen auf Tetrafluorphthalsäureanhydrid bzw. Tetrafluorphthalsäure, einsetzt.

7. Verfahren nach mindestens einem der Ansprüche 1 - 6, dadurch gekennzeichnet, daß man 200 bis 300 mol.-% Zink, bezogen auf Tetrafluorphthalsäureanhydrid bzw. Tetrafluorphthalsäure, einsetzt.

8. Verfahren nach mindestens einem der Ansprüche 1 - 7, dadurch gekennzeichnet, daß man bei Atmosphärendruck, Überdruck oder Unterdruck dehalogeniert.

## Claims

1. A process for the preparation of 3,4,6-trifluorophthalic acid of the formula (1) 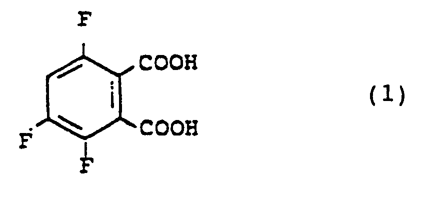 or of the anhydride thereof, which comprises dehalogenating tetrafluorophthalic anhydride of the formula (2) 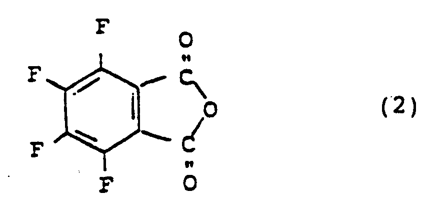 or tetrafluorophthalic acid using zinc in aqueous-alkaline medium at temperatures of 20°C to 160°C and, if appropriate, converting the resulting 3,4,6-trifluorophthalic acid into the anhydride in a known manner by dehydrating it.

2. The process as claimed in claim 1, wherein the dehalogenation is carried out at temperatures of 80°C to 110°C.

3. The process as claimed in at least one of claims 1 and 2, wherein hydroxides, carbonates, hydrogen carbonates or phosphates of alkali metals or alkaline earth metals are used for adjusting the aqueous-alkaline reaction medium.

4. The process as claimed in at least one of claims 1 - 3, wherein the hydroxides, carbonates, hydrogen carbonates or phosphates of alkali metals or alkaline earth metals are employed in amounts such that the pH in the aqueous reaction medium is 7 to 15.

5. The process as claimed in at least one of claims 1 - 4, wherein the hydroxides, carbonates, hydrogen carbonates or phosphates of alkali metals or alkaline earth metals are employed in amounts such that the pH of the aqueous reaction medium is 11 to 14.

6. The process as claimed in at least one of claims 1 - 5, wherein 100 to 500 mol% of zinc, based on tetrafluorophthalic anhydride or tetrafluorophthalic acid, are employed.

7. The process as claimed in at least one of claims 1 - 6, wherein 200 to 300 mol% of zinc, based on tetrafluorophthalic anhydride or tetrafluorophthalic acid, are employed.

8. The process as claimed in at least one of claims 1 - 7, wherein the dehalogenation is carried out under atmospheric pressure, elevated pressure or reduced pressure.

## Revendications

1. Procédé pour la préparation d'acide 3,4,6-trifluorophtalique de formule (1) 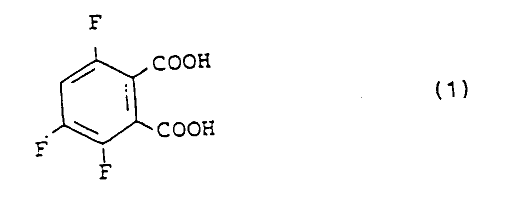 respectivement de son anhydride, caractérisé en ce qu'on déshalogène l'anhydride de l'acide tétrafluorophtalique de formule (2) 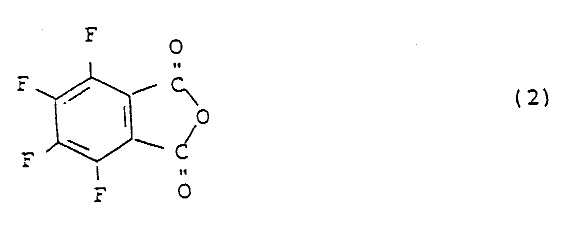 ou l'acide tétrafluorophtalique en milieu aqueux alcalin avec du zinc, à des températures de 20°C à 160°C, et éventuellement on transforme l'acide 3,4,6,-trifluorophtalique obtenu par déshydratation de façon connue, en l'anhydride.

2. Procédé selon la revendication 1, caractérisé en ce qu'on effectue la déshalogénation à des températures de 80°C à 110°C.

3. Procédé selon au moins l'une des revendications 1 et 2, caractérisé en ce que pour l'établissement du milieu réactionnel aqueux alcalin, on utilise des hydroxydes, des carbonates, des bicarbonates ou des phosphates de métaux alcalines ou alcalino-terreux.

4. Procédé selon au moins l'une des revendications 1 à 3, caractérisé en ce qu'on utilise les hydroxydes, les carbonates, les bicarbonates ou les phosphates de métaux alcalins ou alcalino-terreux en de telles quantités que le pH du milieu réactionnel aqueux soit de 7 à 15.

5. Procédé selon au moins l'une des revendications 1 à 4, caractérisé en ce qu'on utilise les hydroxydes, les carbonates, les bicarbonates ou les phosphates de métaux alcalins ou alcalino-terreux en de telles quantités que le pH de milieu réactionnel aqueux soit de 11 à 14.

6. Procédé selon au moins l'une des revendications 1 à 5, caractérisé en ce qu'on utilise de 100 à 500% en moles de zinc par rapport à l'anhydride, respectivement à l'acide tétrafluorophtalique.

7. Procédé selon au moins l'une des revendications 1 à 6, caractérisé en ce qu'on utilise de 200 à 300% en moles de zinc par rapport à l'anhydride de l'acide tétrafluorophtalique, respectivement à l'acide tétrafluorophtalique.

8. Procédé selon au moins l'une des revendications 1 à 7, caractérisé en ce qu'on effectue la déshalogénation sous pression atmosphérique, sous surpression ou sous pression réduite.
